(19) **Europäisches Patentamt**
**European Patent Office**
**Office européen des brevets**

(11) Publication number: **0 204 174 B1**

(12) **EUROPEAN PATENT SPECIFICATION**

(45) Date of publication of patent specification: **09.10.91**

(51) Int. Cl.⁵: **A61M 1/16**

(21) Application number: **86106333.7**

(22) Date of filing: **09.05.86**

(54) **A system for the control of dialysis.**

(30) Priority: **04.06.85 SE 8502757**

(43) Date of publication of application:
**10.12.86 Bulletin 86/50**

(45) Publication of the grant of the patent:
**09.10.91 Bulletin 91/41**

(84) Designated Contracting States:
**BE CH DE FR GB IT LI NL**

(56) References cited:
**EP-A- 0 106 940       EP-A- 0 143 340**
**GB-A- 2 003 274       US-A- 3 946 731**
**US-A- 4 021 341       US-A- 4 486 303**

(73) Proprietor: **GAMBRO AB**
**Post Box 10101**
**S-220 10 Lund(SE)**

(72) Inventor: **Gummesson, Bengt-Ake Göran**
**Korgpilsgränd 4**
**S-230 40 Bara(SE)**
Inventor: **Petersén, Preben Allan**
**Kamrersvägen 46**
**S-237 00 Bjärred(SE)**
Inventor: **Sternby, Jan Peter**
**Sparsnögatan 45**
**S-222 52 Lund(SE)**

(74) Representative: **Boberg, Nils Gunnar Erik**
**Gambro AB Patent Department Box 10101**
**S-220 10 Lund(SE)**

## Description

The present invention relates to a system for the control of dialysis and especially the ultrafiltration in connection therewith, comprising means for the connection of a dialyzer and means for the checking and control of the respective flows on both sides of a membrane included in the dialyzer, and two flow arrangements for the control of one or the other fluid flow, one located before and one after the dialyser, at least one of the two flow arrangements being controllable for the control of the ultrafiltration.

The invention primarily relates to a system for the control of haemodialysis, that is to say for the purifying of the blood of patients with diminished renal function or wholly lacking such function. It will be obvious, however, to those versed in the art that the system in accordance with the invention also may be used for, for example, the control of dialysis in general.

More particularly the invention relates to a further development of the system which is marketed by the Gambro Group, to which the applicant belongs, under the description "Gambro AK-10".

Various details of this system form the subject of, for example, the US patents: 4 122 010, 4 158 034, 4 293 409, 4 194 974 and 4 191 351, these descriptions being included hereby in the present description. Reference is made also to the further development according to EP-A-0 143 340 and the invention according to the GB-A-2 003 274 and the further development of this according to the European patent application published under number EP-A-106 940, and these too are included in the present description. Furthermore, reference is made to EP-A-0 022 922 which describes in more detail the preparation of dialysis fluid from two concentrates, a preparation which is only touched upon in the following description of the present invention.

Known systems of the above mentioned art are disclosed in for instance US-A-3 946 731, US-A-4 486 303 and US-A-4 021 341.

The invention thus relates to a system of the abovementioned type which, more particularly, as defined in the preamble of claim 1 and is characterized by the features specified in the second part of claim 1. By this design a very rapid control is made possible which can be carried out independently of the transmembrane pressure (TMP) which otherwise is frequently used for the control of the ultrafiltration, but which normally is a slow control factor owing, among other things, to the resilience of the membrane in the dialyzer.

Appropriately said pressure measurement is only made in the flow between the pump and the constriction, whilst the pressure on the other side of the contraction preferably is held constant, e.g. at atmospheric pressure. In such a design the measured pressure may be used to control the flow capacity of the pump and this can be done in a fraction of a second. If the voltage of the motor is altered, the time from such an alteration of the voltage up to an alteration of flow may be in the order of magnitude of 0.1 s. As a result the system also can cope with pressure surges which in many systems known previously give rise to an ungovernable ultrafiltration.

The two constant flow arrangements appropriately are of essentially identical design and capacity but individually controllable independently of each other. This naturally simplifies the design at the same time as providing freedom to operate at varying total flow and varying pressure. The control as a whole, naturally, can also be made simpler if identical constant flow arrangements are used.

For reason of safety, means for measuring the pressure in the dialysis fluid flow are also provided, at least on one side of the dialyzer between this and the respective constant flow arrangement for checking and/or controlling the transmembrane pressure (TMP). At the same time, of course the blood pressure on the other side of the membrane is measured.

Appropriately the system in accordance with the invention also comprises means for the measurement of the dialysis fluid flow before and after the dialyzer. This means may be constituted, for example, of a differential measuring arrangement for the direct measurement of the difference in the fluid flow before and after the dialyzer respectively, this difference preferably being adapted so as to be integrated and used for the control of one or of both of the constant flow arrangements. The measuring system itself is described in greater detail in the two aforementioned publications GB-A-2 003 274 and EP-A-0 106 940.

Special advantages are gained if the said differential measuring arrangements are used to counteract slow alterations of the ultrafiltration, caused, for example, by membrane offset, whilst one or both constant flow arrangements are used for counteracting rapid alterations of the ultrafiltration, caused, for example, by accidental pressure surges. As a result an apparently very reliable control of the ultrafiltration is obtained which, moreover, will be substantially independent of accidental pressure surges.

Similar advantages, however, may be obtained by making use instead of the transmembrane pressure measured to counteract slow alterations of the ultrafiltration, caused e.g. by drift of the measuring instrument used, whilst one or both constant flow arrangements are used for counteracting rapid alterations of the ultrafiltration caused, for example, by accidental pressure surges.

In an alternative mode of realization of the subject of the invention the two constant flow arrangements are adapted so as to maintain the same constant flow, at the same time as the ultrafiltration is adapted so as to be controlled through withdrawal of the ultrafiltrate between them with the help of a controllable ultrafiltrate pump.

BRIEF DESCRIPTION OF DRAWINGS

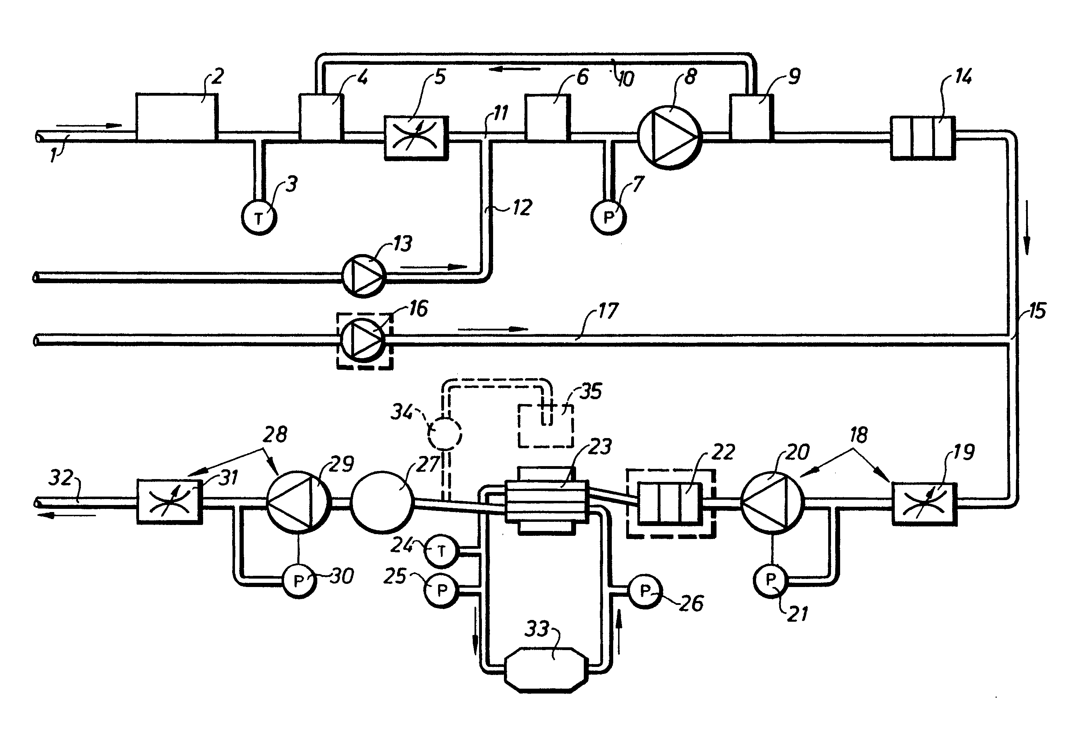

The invention is described in more detail in the following with reference to the attached drawing which, by way of example, shows the most important components of a system in accordance with the invention.

BEST MODE OF CARRYING OUT THE INVENTION

In the system shown as an example in the attached drawing the water is fed via an inlet 1 to a heating device 2 where it is heated. The temperature is then measured in a temperature measuring device 3 before the water is conducted via a return vessel 4 to a constriction 5 and further via a bubble expansion tank 6, a pressure gauge 7 and a pump 8 to a vent tank 9. From the vent tank 9 a return duct 10 leads back to the return vessel 4 for recycling any air or other gases separated. A recycling would be possible instead to the heating vessel 2, but this would then require to be made from a more resistant material since dialysis concentrate is being supplied to the point 11 via the duct 12 with the help of the pump 13. This part of the system corresponds in substantial parts to the system which is described, for example, in the US patents 4 158 034 and 4 293 409. The function of the expansion vessel 6 is described in detail in EP-A-0 143 340. From the vent tank 9 the fluid is passed further via a conductivity measuring cell 14 to another mixing point 15 where further concentrate is supplied possibly with the help of a pump 16 and a duct 17. This is done on the assumption that it is intended to work with so-called two-component-based dialysis concentrate, e.g. of the type which is described in EP-A-0 022 922.

From the mixing point 15 the dialysis fluid is passed through a first constant flow arrangement 18 consisting of a constriction 19, a pump 20 and a pressure gauge 21. Before the constriction device 19 the pressure is substantially at atmospheric pressure as the duct is connected without any major resistance to the vent tank 9. Should it be found in practice that the pressure varies owing to the pump 16, the conductivity meter 14 and the mixing point 15 can be moved to a position before the vent tank.

The pressure measured by the pressure gauge 21 is used for the control of the pump 20, so that a desired constant flow is obtained. After the pump 20 the flow is passed through a conductivity meter 22 and an ultrafiltration monitor 23 via temperature measuring instrument 24 and a pressure gauge 25 to the dialyzer. From the dialyzer the flow is conducted via a pressure gauge 26, the said ultrafiltration monitor 23 and a blood detector 27 to a further constant flow arrangement 28 consisting of a pump 29, a pressure gauge 30 and a constriction device 31. Finally the dialysate is passed to an outlet 32. It is appropriate for the constant flow arrangement 28 to be identical with the arrangement 18. In the constriction 31, though, the pressure drop obtained is from a pressure above atmospheric to the pressure prevailing at the outlet which ought to be kept constant and, for example, may be equal to atmospheric pressure.

The design and the function of the ultrafiltration monitor are described in greater detail in the abovementioned publications GB-A-2 003 274 and EP-A-0 106 940.

Numeral 33 designates the dialyzer which can be connected to the system in accordance with the invention, on the blood side of which the patient is connected up. The latter connection, however, is not shown in the drawing. Finally, the broken lines show how ultrafiltrate can be withdrawn between the two constant flow arrangements 18 and 28 with the help of a pump 34 and possibly be collected in the receiver vessel 35. Hereby the ultrafiltration is appropriately controlled in that the two constant flow arrangements 18 and 28 are adapted to produce identical flows, so that the ultrafiltration can be wholly controlled with the help of the pump 34.

Naturally the invention is not limited merely to the embodiment described above, but to the scope of the following claims. Compare, for example, the publications cited above and out patent applications EP-A-0 204 260 and EP-A-0 208 090 submitted at the same time. Furthermore, it is possible, for example, instead of applying the invention to the dialysis fluid, to obtain similar advantages by arranging the two constant flow arrangements in the blood flow on either side of the dialyzer.

**Claims**

1. A system for the control of dialysis and especially the ultrafiltration in connection therewith, comprising means for the connection of a dialyzer, means for checking and controlling the respective fluid flows on both sides of a membrane included in the dialyzer, and two flow arrangements, comprising a pump (20 or 29) and a flow meter coupled in series (18, 28) for the control of one or the other fluid flow, one located before and one after the dialyzer (33), at least one of the two flow arrangements (18 and/or 28) being controllable for the control of the ultrafiltration, **characterized** in that said flow meter comprises a constriction (19 or 31) and means (21 or 30) for measuring a pressure which is a function of the pressure drop over said constriction said pressure being used as the controlling parameter in order to obtain a desired constant flow through the said constriction -

2. A system in accordance with claim 1, **characterized** in that said pressure measurement is only made in the flow between the pump (20 or 29) and the constriction (19 or 31), whilst the pressure on the other side of the constriction (19 or 31) preferably is kept constant, e.g. at atmospheric pressure.

3. A system in accordance with claim 1, **characterized** in that the measured pressure is used to control the flow capacity of the pump (20 or 29).

4. A system in accordance with anyone of the preceding claims, **characterized** in that the two constant flow arrangements (18,28) are of essentially identical design and capacity, but individually controllable independently of each other.

5. A system in accordance with anyone of the preceding claims, **characterized** by means (25,26) for measuring the pressure in the dialysis fluid flow at least on one side of the dialyzer (33) between this and the respective constant flow arrangement for the checking of TMP (transmembrane pressure).

6. A system in accordance with a anyone of the preceding claims, **characterized** by means (23) for measuring the dialysis fluid flow before as well as after the dialyzer (33).

7. A system in accordance with claim 6, **characterized** in that the said means for the measurement of the dialysis fluid flow is constituted of a differential measuring arrangement (23) for the direct measurement of the difference in the fluid flow before and after the dialyzer (33) respecitvely, this difference preferably being adpated so as to be integrated and used for the control of one or of both of the constant flow arrangements (18,28).

8. A system in accordance with claim 7, **characterized** in that the said differential measuring arrangement (23) is used to counteract slow alterations of the ultrafiltration, e g caused by membrane offset, whilst one or both constant flow arrangements (18 and/or 28) are used for counteracting rapid alterations of the ultrafiltration, e g caused by accidental pressure surges.

9. A system in accordance with claim 5, **characterized** in that the transmembrane pressure measured is used for counteracting slow alterations of the ultrafiltration, e g caused by drift of the measuring instrument used, whilst one or both constant flow arrangements (18 and/or 28) are used for counteracting rapid alterations of the ultrafiltration e g caused by accidental pressure surges.

10. A system in accordance with claim 1, **characterized** in that the two constant flow arrangements are adapted so as to maintain the same constant flow and that the ultrafiltration is adapted so as to be controlled through withdrawal of the ultrafiltrate between them with the help of a controllable ultrafiltrate pump (34).

**Revendications**

1. Système pour le contrôle de la dialyse et en particulier de l'ultrafiltration en relation avec celle-ci, comprenant des moyens pour le raccordement d'un dialyseur, des moyens de vérification et de commande des écoulements de fluide respectifs des deux côtés d'une membrane incorporée dans le dialyseur, et deux dispositifs de circulation (18,28) comprenant une pompe (20 ou 29) et un débitmètre raccordés en série, pour le réglage d'un écoulement d'un fluide ou de l'autre, un dispositif étant placé avant le dialyseur et l'autre étant placé après le dialyseur (33), au moins un des deux dispositifs de circulation (18 et/ou 28) étant réglable pour la commande de l'ultrafiltration, caractérisé en ce que ledit débitmètre comprend un orifice calibré (19 ou 31) et des moyens (21 ou 30) de mesure d'une pression qui est fonction de la perte de charge dans ledit orifice calibré, ladite pression étant utilisée comme paramètre de commande afin d'obtenir un débit constant désiré à travers le dit orifice calibré.

2. Système suivant la revendication 1, caractérisé en ce que ladite mesure de pression est effectuée seulement dans la circulation entre la pompe (20 ou 29) et l'orifice calibré (19 ou 31), tandis que la pression de l'autre côté de l'orifice calibré (19 ou 31) est de préférence maintenue constante, par exemple à la pression atmosphérique.

3. Système suivant la revendication 1, caractérisé en ce que la pression mesurée est utilisée pour régler la capacité de débit de la pompe (20 ou 29).

4. Système suivant l'une quelconque des revendications précédentes, caractérisé en ce que les deux dispositifs de débit constant (18,28) sont de caractéristiques et de capacité sensiblement identiques mais ils sont réglables individuellement et indépendamment l'un de l'autre.

5. Système suivant l'une quelconque des revendications précédentes, caractérisé par des moyens (25,26) de mesure de la pression dans la circulation de fluide de dialyse, au moins d'un côté du dialyseur (33) entre celui-ci et le dispositif de débit constant respectif, pour la vérification de la pression transmembrane (PTM).

6. Système suivant l'une quelconque des revendications précédentes, caractérisé par des moyens (23) de mesure de la circulation de fluide de dialyse, avant et après le dialyseur (33).

7. Système suivant la revendication 6, caractérisé en ce que lesdits moyens de mesure de la circulation de fluide de dialyse sont constitués d'un dispositif de mesure différentielle (23) pour la mesure directe de la différence de débit de fluide avant et après le dialyseur (33) respectivement, cette différence étant de préférence traitée de manière à être intégrée et utilisée pour la commande d'un ou des deux dispositifs de débit constant (18,28).

8. Système suivant la revendication 7, caractérisé en ce que ledit dispositif de mesure différentielle (23) est utilisé pour compenser les variations lentes de l'ultrafiltration, provoquées par exemple par un décalage de membrane, tandis qu'on utilise un des dispositifs de débit constant ou les deux (18 et/ou 28) pour compenser les variations rapides de l'ultrafiltration provoquées par exemple par des pointes de pression accidentelles.

9. Système suivant la revendication 5, caractérisé en ce que la pression transmembrane mesurée est utilisée pour compenser les variations lentes de l'ultrafiltration, provoquées par exemple par une dérive de l'instrument de mesure utilisé, tandis qu'on utilise un des dispositifs de débit constant ou les deux(18 ou/et 28) pour compenser les variations rapides de l'ultrafiltration, provoquées par exemple par des pointes de pression accidentelles.

10. Système suivant la revendication 1, caractérisé en ce que les deux dispositifs de débit constant sont prévus pour maintenir le même débit constant, et en ce que l'ultrafiltration est prévue pour être réglée par extraction de l'ultrafiltrat entre les dispositifs à l'aide d'une pompe d'ultrafiltrat réglable (34).

**Patentansprüche**

1. System für die Kontrolle der Dialyse und besonders der Ultrafiltration in Verbindung mit ihr mit Einrichtungen zur Verbindung eines Dialysegerätes, Einrichtungen zur Überwachung und Kontrolle der betreffenden Fließmittelströme auf beiden Seiten der in dem Dialysegerät enthaltenen Membran und zwei Fließanordnungen (18, 28) mit einer Pumpe (20 oder 29) und einem Flußmeßgerät, die in Reihe miteinander verbunden sind, für die Kontrolle eines oder des anderen Fließmittelflusses, wobei eine vor und eine nach dem Dialysegerät (33) angeordnet ist und wobei wenigstens eine der beiden Fließanordnungen (18 und/oder 28) für die Kontrolle der Ultrafiltration steuerbar ist, **dadurch gekennzeichnet,** daß das Flußmeßgerät eine Einschnürung (19 oder 31) und Einrichtungen (21 oder 30) für die Messung eines Druckes hat, der eine Funktion des Druckabfalles bei dieser Einschnürung ist, wobei der Druck als der Kontrollparameter verwendet wird, um einen erwünschten konstanten Fluß durch die Einschnürung zu erhalten.

2. System nach Anspruch 1, **dadurch gekennzeichnet,** daß die Druckmessung nur in dem Fluß zwischen der Pumpe (20 oder 29) und der Einschnürung (19 oder 31) gemacht wird, während der Druck auf der anderen Seite der Einschnürung (19 oder 31) vorzugsweise konstant, z. B. auf Atmosphärendruck, gehalten wird.

3. System nach Anspruch 1, **dadurch gekennzeichnet,** daß der gemessene Druck verwendet wird, um die Fließkapazität der Pumpe (20 oder 29) zu steuern.

4. System nach einem der vorausgehenden Ansprüche, **dadurch gekennzeichnet,** daß die beiden konstanten Fließanordnungen (18, 28) im wesentlichen identische Konstruktion und Kapazität haben, aber unabhängig voneinander einzeln steuerbar sind.

5. System nach einem der vorausgehenden Ansprüche, **gekennzeichnet durch** Einrichtungen (25, 26) zur Messung des Druckes in dem Dialyseflüssigkeitsfluß wenigstens auf einer Seite des Dialysegerätes (33) zwischen ihm und der betreffenden konstanten Fließanordnung für die Überwachung des TMP (Transmembrandruckes).

6. System nach einem der vorausgehenden Ansprüche, **gekennzeichnet durch** Einrichtungen (23) zur Messung des Dialyseflüssigkeitsflusses vor und nach dem Dialysegerät (33).

7. System nach Anspruch 6, **dadurch gekennzeichnet,** daß die Einrichtungen für die Messung des Dialyseflüssigkeitsflusses aus einer Differentialmeßanordnung (23) für die direkte Messung des Unterschiedes des Flüssigkeitsflusses vor bzw. nach dem Dialysegerät (33) bestehen, wobei dieser Unterschied vorzugsweise so angepaßt ist, daß er integriert und für die Kontrolle einer oder beider der konstanten Fließanordnungen (18, 28) verwendet werden kann.

8. System nach Anspruch 7, **dadurch gekennzeichnet,** daß die Differntialmeßanordnung (23) verwendet wird, langsamen Änderungen der Ultrafiltration, wie sie beispielsweise durch Membranabweichungen verursacht werden, entgegenzuwirken, während eine oder beide der konstanten Fließanordnungen (18 und/oder 28) verwendet werden, um schnellen Änderungen der Ultrafiltration, wie sie beispielsweise durch gelegentliche Durckstöße verursacht werden, entgegenzuwirken.

9. System nach Anspruch 5, **dadurch gekennzeichnet,** daß der gemessene Transmembrandruck verwendet wird, langsamen Änderungen der Ultrafiltration, wie sie beispielsweise durch eine Abweichung des verwendeten Meßinstrumentes verursacht werden, entgegenzuwirken, während eine oder beide der konstanten Fließanordnungen (18 und/oder 28) verwendet werden, um raschen Änderungen der Ultrafiltration, wie sie beispielsweise durch gelegentliche Druckstöße verursacht werden, entgegenzuwirken.

10. System nach Anspruch 1, **dadurch gekennzeichnet,** daß die beiden konstanten Fließanordnungen so ausgebildet sind, daß sie den gleichen konstanten Fluß aufrechterhalten, und daß die Ultrafiltration so ausgebildet ist, daß sie durch Abziehen des Ultrafiltrats zwischen ihnen mit Hilfe einer steuerbaren Ultrafiltratpumpe (34) gesteuert wird.